# EUROPÄISCHE PATENTSCHRIFT

(11) **EP 1 563 419 B1**
(45) Veröffentlichungstag und Bekanntmachung des Hinweises auf die Patenterteilung: **09.09.2020**
(21) Anmeldenummer: 02794505.4
(22) Anmeldetag: 17.07.2002
(51) Int. Cl.: G06Q 99/00, E04B 1/72, C12N 1/14

(54) **VERFAHREN ZUR VORHERSAGE VON SCHIMMELPILZBILDUNG**
METHOD FOR PREDICTING THE FORMATION OF MOULD FUNGI
PROCEDE PERMETTANT DE PREVOIR LE DEVELOPPEMENT DE MOISISSURES

(30) Priorität: 02.08.2001 DE 10137889
(43) Veröffentlichungstag der Anmeldung: 17.08.2005
(73) Patentinhaber: Fraunhofer-Gesellschaft zur Förderung der angewandten Forschung e.V., 80686 München (DE)
(72) Erfinder: SEDLBAUER, Klaus, 83607 Holzkirchen (DE); KRUS, Martin, 81549 München (DE)
(74) Vertreter: Pfenning, Meinig & Partner mbB
(86) Internationale Anmeldenummer: PCT/EP2002/007951
(87) Internationale Veröffentlichungsnummer: WO 2003/014986

(56) Entgegenhaltungen:
- C M Johnstone ET AL: "Development of a Simulation Tool for Mould Growth Prediction in Buildings", Fifth International IBPSA Conference, International Building Performance Simulation Association : proceedings, 10 September 1997 (1997-09-10), XP055672899, Retrieved from the Internet: URL:http://citeseerx.ist.psu.edu/viewdoc/d ownload?doi=10.1.1.627.8341&rep=rep1&type= pdf [retrieved on 2020-03-02]
- A. Hukka ET AL: "A mathematical model of mould growth on wooden material", WOOD SCIENCE AND TECHNOLOGY., vol. 33, no. 6, 7 December 1999 (1999-12-07), pages 475-485, XP055672900, DE ISSN: 0043-7719, DOI: 10.1007/s002260050131

## Beschreibung

Die vorliegende Erfindung betrifft ein Verfahren zur Vorhersage von Schimmelpilzbildung sowie dessen Anwendungen zur Optimierung von Bauten und Bauprodukten bzw. von Lebensmitteln und dergleichen.

Schimmelpilzbefall, insbesondere an Innenoberflächen von Außenbauteilen, aber auch an deren Stellen auf und innerhalb von Bauteilen hat in letzter Zeit von sich reden gemacht. Seine Beseitigung bzw. Vermeidung führt nicht nur zu erheblichen Sanierungskosten. Schimmelpilz kann auch die Gesundheit der Bewohner gefährden. Zwar besteht die Möglichkeit, durch Biozide oder ähnliche Mittel Schimmelpilzbefall in Räumen zu vermindern oder über gewisse Zeit zu verhindern. Allerdings kann eine Gesundheitsgefährdung durch diese Produkte nicht ausgeschlossen werden. Zur Vermeidung von Schimmelpilzbildung in Gebäuden muß deshalb eine Verhinderungsstrategie entwickelt werden, die von den Wachstumsvoraussetzungen für Schimmelpilze ausgeht und die komplexen bauphysikalischen instationären Vorgänge berücksichtigt.

Es hat sich gezeigt, daß die drei wesentlichen Wachstumsvoraussetzungen "Temperatur, Feuchte und Substrat" über eine bestimmte Zeitperiode simultan vorhanden sein müssen, um Pilzwachstum zu ermöglichen. In der Literatur wurde zunächst meist nur die relative Feuchte als einziges Kriterium genannt. Mittlerweile gibt es auch Angaben über relative Feuchten in Abhängigkeit von der Temperatur, bei deren Überschreitung Schimmelpilzbildung auftreten kann. Diese Kennlinien erlauben in der Regel aber keine Differenzierung des Einflusses von Substrat, Baustoff oder der Verschmutzung. Die derzeit üblichen Bewertungsmethoden für Schimmelpilzbildung erlauben keine oder eine nur indirekte Berücksichtigung instationärer Randbedingungen.

Ausgehend von diesem Stand der Technik ist es daher Aufgabe der vorliegenden Erfindung ein Verfahren und Verwendungen derartiger Verfahren zur Verfügung zu stellen, mit denen die Schimmelpilzbildung auf oder in einem Gegenstand zuverlässig vorhergesagt werden kann.

Diese Aufgabe wird durch das Verfahren nach Anspruch 1 sowie die Verwendungen dieses Verfahrens nach Anspruch 14 gelöst. Vorteilhafte Weiterbildungen des erfindungsgemäßen Verfahrens werden in den entsprechenden abhängigen Ansprüchen gegeben.

Das erfindungsgemäße biohygrothermische Verfahren ermöglicht die Vorhersage von Schimmelpilzbildung auf Basis der drei oben genannten biologischen Wachstumsvoraussetzungen von Schimmelpilzen, nämlich der Temperatur, der Feuchte und des Substrates, bei instationären Randbedingungen. Es kann für alle Arten von Bauteilen und Baustoffen einschließlich Holz, aber beispielsweise auch bzgl. der Schimmelbildung auf Lebensmittel eingesetzt werden. Das Verfahren besteht dabei aus zwei aufeinander aufbauenden Schritten, nämlich der Erzeugung geeigneter Isoplethen und der Anwendung dieser Isoplethen auf ein instationären biohygrothermisches Modell des zu untersuchenden Bauteiles. Es ermöglicht die Ermittlung der Sporenauskeimungszeiten und des Myzelwachstums, wobei auch der Substrateinfluß bei der Vorhersage der Schimmelpilzbildung berücksichtigt wird. Das Isoplethensystem beschreibt dabei die hygrothermischen Wachstumsvoraussetzungen eines Pilzes und besteht aus einem von der Temperatur und der relativen Feuchte abhängigen Kurvensystem, den sog. "Isoplethen", die im Falle der Vorhersage von Sporenkeimung Sporenauskeimungszeiten, im Falle der Beschreibung des Myzelwachstums Wachstum pro Zeiteinheit kennzeichnen.

Da sich zwischen einzelnen Pilzspezies signifikante Unterschiede des Isoplethensystems ergeben, wurde bei der Entwicklung des für das erfindungsgemäße Verfahren für den Bereich der Baustoffe gültigen Isoplethensystems vorteilhafterweise nur Pilze berücksichtigt, die in Gebäuden auftreten und gesundheitsbeeinträchtigend sind. Für diese etwas mehr als 150 Spezies die beide Merkmale erfüllen, wurden quantitative Angaben in Form von Isoplethen zu den Wachstumsparametern Temperatur und Feuchte erstellt. Dabei werden vorteilhafterweise zur Unterscheidung der Lebensphasen der Schimmelpilze die Isoplethen getrennt für die Sporenauskeimung und das Myzelwachstum dargestellt. Vorteilhafterweise erfolgt weiterhin eine Differenzierung der Schimmelpilze nach der von ihnen ausgehenden Gesundheitsgefährdung in Form sogenannter Gefährdungsklassen, welche vorteilhafterweise folgendermaßen definiert werden können:
A. Pilz oder Stoffwechselprodukte sind erheblich gesundheitsgefährdend und dürfen in einem Wohnraum nicht auftreten.
B. Pilz oder Stoffwechselprodukte sind bei längerer Exposition in Räumen gesundheitsgefährdend (d.h. pathogen) oder besitzen ein allergenes Potential.
C. Pilz ist nicht gesundheitsgefährdend, ein Bewuchs führt aber ggf. zu wirtschaftlichem Schaden.

Da sich bei der Einteilung der Pilze in die drei Gefährdungsklassen die Werte für Klasse C nur unwesentlich von B unterscheiden, reicht es vorteilhafterweise aus, im Isoplethenmodell nur die die Gefährdungsklassen A und B/C zu unterscheiden. Die Isoplethensysteme werden vorteilhafterweise zur Beurteilung der Sporenauskeimung und des Myzelwachstums entwickelt und basieren beispielsweise auf meßtechnisch erfaßten biologischen Daten und berücksichtigen die Wachstumsvoraussetzung aller Pilze einer jeweiligen Gefährdungsklasse. Die sich dabei ergebenden untersten Grenzen möglicher Pilzaktivität werden LIM (Lowest Isopleth for Mould) genannt. Hierzu werden zunächst für die Gefährdungsklassen die Wachstumsvoraussetzungen von Schimmelpilzen in Abhängigkeit von Temperatur und relativer Feuchte für optimalen Nährboden angegeben. Um den Einfluß des Substrats, also des Untergrundes oder ggf. eventueller Untergrundverunreinigungen, auf die Schimmelpilzbildung berücksichtigen zu können, werden vorteilhafterweise Isoplethensysteme für zwei Substratgruppen (Grenzkurve LIM_{BAU}) vorgeschlagen, die aus experimentellen Untersuchungen abgeleitet werden.

Für die Erstellung von Isoplethensysteme für verschiedene Substratgruppen werden vorteilhafterweise folgende vier Substratgruppen, die unterschiedlichen Untergründen entsprechen, betrachtet:

| | |
|---|---|
| Substratgruppe 0: | Optimaler Nährboden (z.B. Vollmedien), |
| Substratgruppe I: | biologisch verwertbare Substrate, wie z.B. Tapeten, Gipskarton, Bauprodukte aus gut abbaubaren Rohstoffen, Material für dauerelastische Fugen, |
| Substratgruppe II: | Baustoffe mit porigem Gefüge, wie z.B. Putze, mineralische Baustoffe, manche Hölzer sowie Dämmstoffe, die nicht unter Substratgruppe 1 fallen, |
| Substratgruppe III: | Baustoffe, die weder abgebaut werden können noch Nährstoffe enthalten. |

Es genügt dabei bereits ein eigenes Isoplethensystem nur für die mit 0, I und II bezeichneten Gruppen, wobei für die Substratgruppe 0 die Isoplethen für optimalen Nährboden gelten. Die Substratgruppe 0 wird beispielsweise für Lebensmittel wie Brot angenommen. Für die Substratgruppe III wird kein Isoplethensystem benötigt, da davon ausgegangen werden kann, daß ohne Verschmutzung Schimmelpilzbildung nicht auftreten kann. Im Fall einer starken Verschmutzung der Untergründe der Substratgruppen II und III sollte stets die Substratgruppe I zugrunde gelegt werden. Prinzipiell sollte dabei vorteilhafterweise bei dem erfindungsgemäßen Verfahren für die Festlegung der relevanten Substratgruppe immer vom ungünstigsten Fall ausgegangen werden, also das Verfahren im Hinblick auf eine Vermeidung von Schimmelpilzen stets auf der sicheren Seite geführt werden.

Vorteilhafterweise wurden vier Isoplethensysteme jeweils für Sporenauskeimung und Myzelwachstum entwickelt, die für eine ganze Gruppe von Schimmelpilzen gelten und neben optimalem Nährboden auch unterschiedliche Substrate berücksichtigen, nämlich:
a) Isoplethensysteme für die Gefährdungsklasse B/C (LIM B/C). Diese Systeme beziehen sich auf biologische Vollmedien als Nährboden und besitzen daher die anspruchslosesten Wachstumsvoraussetzungen aller Isoplethensysteme, also die niedrigsten Werte für die relative Feuchte. Sie bilden für alle in Gebäuden auftretenden Schimmelpilze die Wachstumsgrenze. Dies bedeutet, daß auch Schimmelpilzbildung der Gefährdungsklasse A ausgeschlossen ist, d.h. A + B + C, wenn für die Klasse B/C die Wachstumsvoraussetzung nicht erfüllt ist.
b) Isoplethensysteme für die Gefährdungsklasse A (LIM A). Analog zu a), nur für alle Pilze der Gefährdungsklasse A gültig,
c) Isoplethensysteme für Substratgruppe I (LIM_{BAU} I). Gelten für alle im Bau auftretenden Schimmelpilze und beziehen sich nicht auf Vollmedium, sondern auf Materialien in Substratgruppe I als Substrat,
d) Isoplethensysteme für Substratgruppe II (LIM_{BAU} II). Analog zu c), nur für alle Materialien, die in Substratgruppe II fallen gültig.

Um die Wirkungsweise der wesentlichen Einflußgröße auf die Auskeimung der Sporen, nämlich die bei bestimmten Temperaturen verfügbare Feuchte, bauphysikalisch beschreiben zu können, wurde ein neuartiges biohygrothermisches Modell entwickelt. Dieses ist in der Lage, den Feuchtehaushalt einer Spore in Abhängigkeit von instationären Randbedingungen rechnerisch zu ermitteln, also auch ein zwischenzeitliches Austrocknen der Pilzsporen zu berücksichtigen. Dieses instationäre biohygrothermische Verfahren beruht auf dem Grundgedanken, daß eine Pilzspore wegen der in ihr vorhandenen Stoffe ein gewisses osmotisches Potential besitzt, mit dessen Hilfe Wasser aus der Umgebung aufgenommen werden kann. Dieses Potential wird rechnerisch mit Hilfe einer Feuchtespeicherfunktion beschrieben. Die Feuchteaufnahme der Spore durch die Sporenwand hindurch wird im Modell mittels eines Diffusionsansatzes erfaßt.

Diese Vereinfachung ist gerechtfertigt, da die Feuchteaufnahme aufgrund der geringen geometrischen Größe der Schimmelpilzspore stets isotherm abläuft. Für die Sporenwand wird dabei ein feuchteabhängiges s_{d}-Wert angesetzt, der durch Vergleich der berechneten mit den in den vorliegenden Isoplethensystemen fixierten Sporenauskeimungszeiten iterativ bestimmt wurde. Ist ein bestimmter Wassergehalt im Sporeninneren vorhanden, der den Beginn des Stoffwechsels zuläßt, kann dann der Pilz unabhängig von äußeren Bedingungen seinen Stoffwechsel selbst regulieren. Der kritische Wassergehalt (Grenzwassergehalt), ab dem die biologische Aktivität einsetzt, darf im vorhergehenden Verfahren jedoch erst gar nicht überschritten werden. Dieser Grenzwassergehalt wird mit Hilfe der beschriebenen Isoplethensysteme für Sporenauskeimung festgelegt, indem temperaturabhängig aus den entsprechenden LIM-Kurven die tiefste relative Feuchte ablesbar ist, bei der Sporenauskeimung stattfindet. Mit Hilfe der für das Sporeninnere zugrunde gelegten Feuchtespeicherfunktion wird dann der in der Spore sich einstellende Wassergehalt errechnet und dann mit dem Grenzwassergehalt verglichen.

Zur Berücksichtigung möglicher Substrateinflüsse können die s_{d}-Werte der Sporenwand und die Feuchtespeicherfunktion so angepaßt werden, daß die unter stationären Bedingungen mit dem erfindungsgemäßen biohygrothermischen Modell ermittelten Sporenauskeimungszeiten denjenigen in den Isoplethensystemen der Substratgruppen 0, I und II entsprechen. Durch dieses Anpassen kann eine Modellspore definiert werden, die für alle drei Substratgruppen Gültigkeit besitzt. Ferner können bei der Festlegung der substratabhängigen Grenzwassergehalte die LIM-Kurven in den Isoplethensystemen der entsprechenden Substratgruppen verwendet werden.

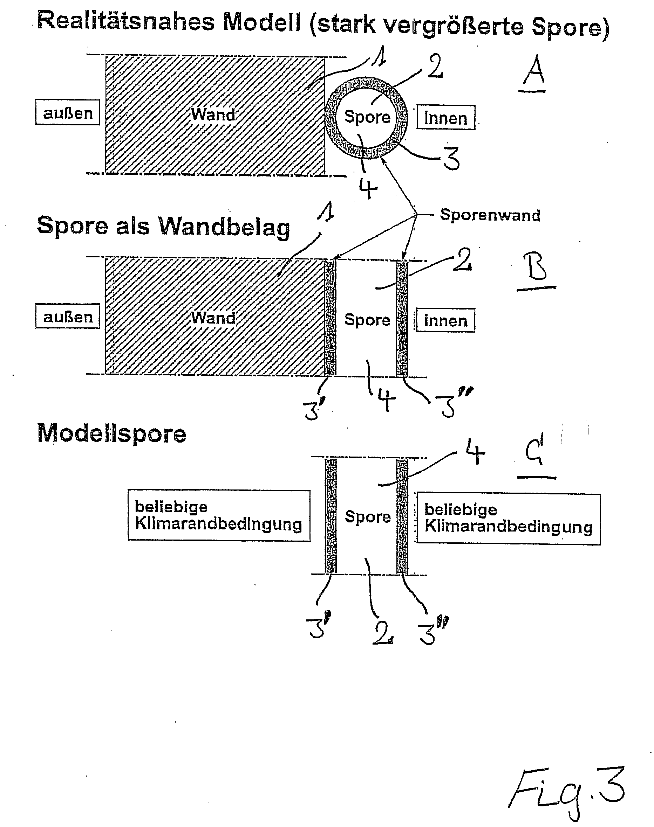

Die im Bau auftretenden instationären Bedingungen für Temperatur und relative Feuchte werden erfindungsgemäß beispielsweise mit einem Berechnungsverfahren zur Ermittlung des instationären Wärme- und Feuchtetransports für ein- und zweidimensionale Baukonstruktionsgeometrien gewonnen oder stammen aus Messungen. Die Beurteilung der Sporenauskeimung erfolgt dabei auf Basis des an der Oberfläche auftretenden Mikroklimas. Fig. 3 zeigt eine quasi reale, vergrößerte Spore.

In den Fign. 3A bis C ist mit dem Bezugszeichen 1 die Wand bezeichnet, auf der die Schimmelpilzspore sitzt und deren Risiko für Schimmelpilzbildung untersucht wird. Mit dem Bezugszeichen 2 ist die Spore bezeichnet, die eine Sporenwand 3 und ein Sporeninneres 4 aufweist. Mit 3', 3'' sind in den Fign. 3B und 3C die modellhaften Sporenwände bezeichnet.

Die Spore 2 kann man sich vorstellen als Kugel mit einer Sporenwand 3. Die reale Spore berührt den Baustoff 1, d.h. die hygrothermischen Randbedingungen an dieser Oberfläche beeinflussen die feuchtetechnischen Vorgänge in der Spore 2. Allerdings beeinflußt die Spore 2 aufgrund ihrer geringen Abmessungen sicherlich nicht die bauphysikalischen Randbedingungen im Bereich der Baustoffoberfläche 1. Daher ist es nicht sinnvoll, eine Gesamtmodellierung, d.h. den Bauteilaufbau mit der Spore 2 als Wandbelag, wie ihn Bild 3B gezeigt, zu verwenden. Ein derartiges Rechenmodell mit der Spore 2 als Schicht vor einem Bauteil 1 würde sogar zu fehlerhaften Ergebnissen führen, da die Spore 2 einen zusätzlich hohen, unrealistischen Diffusionswiderstand darstellen würde. Daher wird vorteilhafterweise die Spore 2 wie in Bild 3 unten als von der Wand 1 unabhängig angenommen. Damit können beliebige Temperatur- und Feuchteverläufe als Klimarandbedingungen für das biohygrothermische Modelle verwendet werden.

Mit dem erfindungsgemäßen Verfahren steht nun ein Planungsinstrument zur Verfügung, um die Schimmelpilzbildung vorherzusagen, das von Bauphysikern und im Baubereich tätigen Beratungsbüros genutzt werden kann. Damit können Auslegungen von Bauprodukten und Lüftungsanlagen durchgeführt werden. Ferner sind Planungsleistungen zu Sanierungsmaßnahmen und deren richtige Ausführung möglich. Weiterhin sind Aussagen zum optimalen Betrieb von Lüftungsanlagen unter Einsatz dieses Verfahrens und ein optimaler Betrieb derartiger Lüftungsanlagen möglich. Die Schimmelpilzbildung kann dabei sowohl an Innenwandoberflächen (zum Raum hin) im Bauteilinneren selbst sowie in Lüftungskanälen oder an Außenfassaden mit dem erfindungsgemäßen Verfahren erfolgen.

Vorteilhaft an dem erfindungsgemäßen Verfahren ist nun, daß erstmals eine Vorhersage der Schimmelpilzbildung instationär erfolgen kann. Die rechnerische Prognose der Schimmelpilzbildung ermöglicht die Behandlung von Fragestellungen, die bislang weder mit einfachen Abschätzungen noch mit sinnvollem meßtechnischen Aufwand beantwortet werden konnten. Zum Beispiel war die experimentelle Bestimmung des hygrothermischen Verhaltens von sanierten Außenwandkonstruktionen und die Bewertung der Gefahr einer wiederkehrenden mikrobiellen Besiedelungsgefahr nach der Sanierung bislang nicht möglich. Parameterstudien zur Wahl der richtigen Konstruktion können mit dem biohygrothermischen Verfahren nunmehr relativ einfach durchgeführt werden. Ferner ist auch die Bewertung von innenliegenden Konstruktionsteilen problemlos. Gerade diese einfachen und kostengünstigen Merkmale lassen bei der Planung von Bauvorhaben oder bei Sanierungsmaßnahmen für die Zukunft einen breiten Einsatz des neuen Verfahrens zur Vorhersage von Schimmelpilzbildung erwarten.

Im folgenden werden einige Beispiele erfindungsgemäßer Verfahren und deren Verwendungen beschrieben werden.

Es zeigen
- Fig. 1: in den Teilbildern A und B die Isoplethensysteme für die Sporenauskeimung für alle Pilze der Substratgruppen I (Fig. 1A) und II (Fig. 1B);
- Fig. 2: das Isoplethensystem für die Sporenauskeimungszeiten für alle Pilze der Gefährdungsklasse A (Fig. 2A) und B/C (Fig. 2B);
- Fig. 3: verschiedene Sporenmodelle;
- Fig. 4: die berechneten Zeitverläufe von Temperatur (Fig. 4A) und relativer Feuchte (Fig. 4B) sowie des Sporenwassergehaltes (Fig. 4C) an verschiedenen Stellen des Außenputzes einer Außenwand.

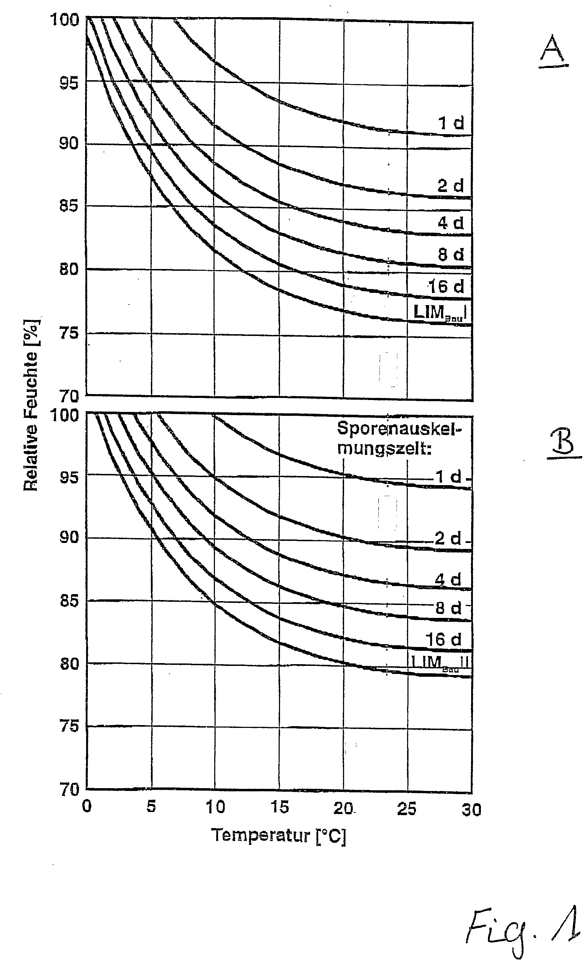

Fig. 1 zeigt in den Teilbildern A und B ein verallgemeinertes Isoplethensystem für die Sporenauskeimung, das für alle Pilze der Substratgruppe I (Fig. 1A) und II (Fig. 1B) gilt. Die Angaben in Tagen bedeuten dabei die Sporenauskeimungszeiten. Unterhalb der mit LIM_{BAU} bezeichneten Kurve ist auf Baustoffen der entsprechenden Gruppe mit keiner biologischen Aktivität, d.h. keiner Sporenauskeimung, zu rechnen.

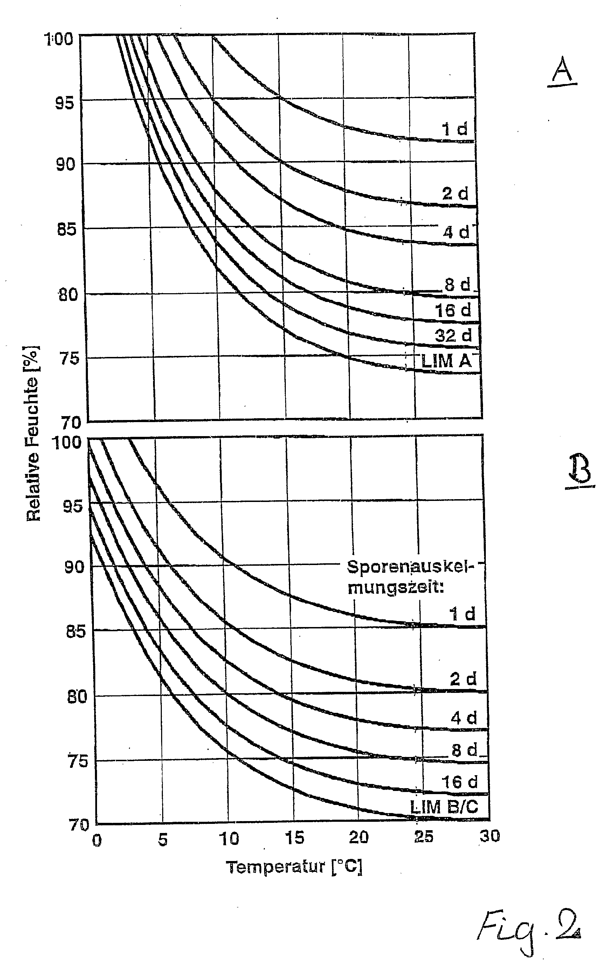

Fig. 2 zeigt in ihren Teilbildern ein verallgemeinertes Isoplethensystem für die Sporenauskeimung das für alle Pilze der Gefährdungsklasse A (Fig. 2A) und B/C (Fig. 2B) gilt. Die Lage der mit LIM bezeichneten Kurve (LIM = Lowest Isopleth for Mould) stellt die unterste Grenze der biologischen Aktivität in einer Gefährdungsklasse dar. Die angegebene Zahl der Tage charakterisiert die Zeitdauer, nach welcher erste Aufkeimung bei der gewählten Temperatur und der gewählten Feuchte auftreten.

Fig. 3 zeigt eine schematisierte Vergleichsdarstellung einer Spore auf einer Wand (Fig. 3A), einer Spore als Wandbelag (Fig. 3B) und einer erfindungsgemäß betrachteten Modellspore (Fig. 3C).

Das Verhältnis zwischen Sporendurchmesser zur Wanddicke (30 cm) beträgt rund 1:100.000. Die reale Spore berührt den Baustoff, d.h. die hygrothermischen Randbedingungen an dieser Oberfläche beeinflussen die feuchtetechnischen Vorgänge in der Spore. Allerdings beeinflußt die Spore aufgrund ihrer geringen Abmessungen nicht die bauphysikalischen Randbedingungen im Bereich der Baustoffoberfläche. Daher wird keine Gesamtmodellierung, gemäß Bauteilaufbau mit der Spore als Wandbelag (Fig. 3B) verwendet, sondern eine Von, der Wand unabhängige "Modellspore" (Fig. 3C). Damit können beliebige Verläufe der Temperatur und relativen Feuchte als Klimarandbedingungen bei biohygrothermischen Berechnungen berücksichtigt werden.

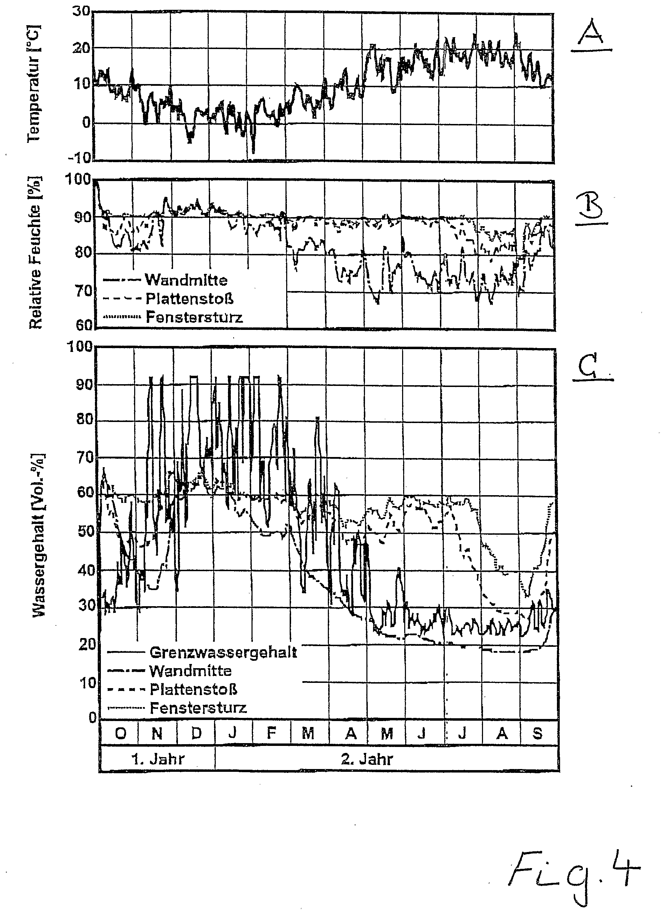

Fig. 4 zeigt beispielhaft berechnete Zeitverläufe von Temperatur (Fig. 4A) und relativer Feuchte (Fig. 4B) sowie des Verlaufs des Sporenwassergehaltes (Fig. 4C) an verschiedenen Stellen des Außenputzes einer Außenwand. Dabei wurden die folgenden Daten und Randbedingungen zugrunde gelegt:
Der Wandaufbau besteht aus WDVS auf Beton als Wandbildner. Als untersuchte Stellen wurde ein ungestörter Bereich ohne Schimmelpilzbefall, ein Plattenstoß des WDVS (Wärmedämpferbundsystems) mit Schimmelpilzbefall sowie ein Fenstersturz mit Schimmelpilzbefall betrachtet. In Fig. 4C ist weiterhin der Verlauf des Grenzwassergehaltes mit eingezeichnet. Er gilt für alle Wandstellen und zeigt ab wann Sporenkeimung einsetzen wird.

Diese Figur schildert die Situation an den Außenfassaden einer Wohnanlage, die im Sommer bis Herbst fertiggestellt wurde. Es zeigte sich nach kurzer Zeit ein sichtbarer biologischer Aufwuchs. Vor allem war im Bereich des Fenstersturzes ein flächiger Schimmelpilzbefall zu erkennen (deutliche Verfärbung). In den Wandflächen in Wandmitte treten vorwiegend kreisförmige Befallsmuster auf. An diesen Stellen wurden die Dämmstoffplatten aus Polystyrol-Hartschaum nicht auf Stoß verlegt, sondern es verblieb zwischen ihnen ein Spalt von etwa drei Millimetern. Dieser Spalt war durchgängig bis auf den darunterliegenden Beton. Der kreisförmige Pilzbefall befand sich etwa im Bereich des Stoßkreuzes von vier Dämmstoffplatten.

Zur Beurteilung der Schimmelpilzbildung wurde der Feuchtehaushalt im Bereich der Fensterstürze sowie des Luftspaltes zwischen den Dämmplatten ermittelt. In Fig. 4 ist für das erste Jahr der Verlauf der Temperatur (Fig. 4A) und der relativen Feuchte im Außenputz (Fig. 4B) an der Stelle des Plattenstoßes (gestrichelte Linie) und des Fenstersturzes (gepunktete Linie) im Vergleich zum Putz im ungestörten Bereich, also in Wandmitte (punkt-gestrichelte Linie) dargestellt. Außer im Zeitraum von Mitte November bis Mitte Januar unterscheiden sich die Verläufe in den beiden unterschiedlichen Stellen wesentlich. Während am Putz (Substratklasse II) die relative Feuchte in Wandmitte ab diesem Zeitpunkt aufgrund der steigenden Außenluft- und damit der Putztemperaturen (Fig. 4A) sinkt, bleibt sie an der Fehlstelle (Plattenstoß) bis etwa Mitte Juli bei annähernd 90 %. Im Bereich des Fenstersturzes ergeben sich im Vergleich zur Fehlstelle im Putz noch höhere Feuchten (nicht in Fig.4 dargestellt). Nimmt man diese Klimadaten als Randbedingungen für Berechnungen mit dem biohygrothermischen Modell, so ist in Fig. 4C zu erkennen, daß die Wassergehalte in den sich auf dem Putz befindlichen Modellsporen im Zeitraum von Mitte November bis Mitte Januar ebenfalls etwa gleich verlaufen, sich sonst aber in Wandmitte geringere Werte ergeben. Die starken Schwankungen im Verlauf des Grenzwassergehalts liegen an den großen Temperaturänderungen an einer Außenfassade. Die Maxima der Grenzwassergehalte bei Temperaturen etwa 0 °C entsprechen der "freien Wassersättiung" der Spore und liegen temperaturunabhängig bei etwa 92 Vol.-%.

Der Wassergehalt in den Sporen in Wandmitte liegt, abgesehen von den Anfangswerten der ersten zwei Wochen nach Fertigstellung, stets unter dem Grenzwassergehalt. Das bedeutet, daß in Wandmitte kein Pilzwachstum auftreten sollte, was auch mit den Beobachtungen am Objekt und Erfahrungen aus der Praxis übereinstimmt. Anders bei den Putzen im Bereich des Fenstersturzes und bei den Fehlstellen an den Plattenstößen. Im Bereich des Fenstersturzes wird der Grenzwassergehalt in den ersten sechs Wochen nach Baufertigstellung und ab April überschritten. Wie auch am Objekt beobachtet, ergibt sich bereits im Frühjahr ein großflächiger Pilzbewuchs. Beim Plattenstoß ergeben sich etwas geringere Überschreitungen des Grenzwassergehaltes, was zu einem etwas geringeren Befall führt.

## Patentansprüche

1. Verfahren zur Vorhersage von Schimmelpilzbildung auf einem Gegenstand, beispielsweise einem Bauteil, indem experimentell und/oder mit einem Computer bestimmte biologische Auskeimungsbedingungen und/oder Wachstumsvoraussetzungen für einen oder mehrere Schimmelpilze mit den auf dem Gegenstand auftretenden experimentell und/oder mit einem Computer bestimmten hygrothermischen Bedingungen verglichen werden und daraus bestimmt wird, ob die Auskeimungsbedingungen und/oder Wachstumsvoraussetzungen auf dem Gegenstand gegeben sind, mit den folgenden Schritten:
- Bestimmung des Temperatur- und relativen Feuchteverlaufs auf dem Gegenstand,
- Bestimmung des Wassergehalts einer Spore auf dem Gegenstand und
- Bestimmung, ob der Wassergehalt der Spore im Laufe der Zeit den für die Auskeimung und/oder Wachstum erforderlichen Wassergehalt (Grenzwassergehalt) erreicht oder überschreitet.

2. Verfahren nach dem vorhergehenden Anspruch, **dadurch gekennzeichnet, dass** zur Bestimmung des erforderlichen Wassergehalts mindestens eine Schar von Isoplethen bezüglich Auskeimung und/oder Wachstum von Schimmelpilzen in Abhängigkeit von Temperatur und Feuchte ermittelt wird.

3. Verfahren nach dem vorhergehenden Anspruch, **dadurch gekennzeichnet, dass** eine Schar von Isoplethen für jede einzeln oder mehrere gemeinsam der folgenden Pilzspezies bestimmt wird: a) alle Pilze, b) Pilze, die in Gebäuden auftreten und gesundheitsgefährdend sind.

4. Verfahren nach einem der beiden vorhergehenden Ansprüche, **dadurch gekennzeichnet, dass** eine Schar von Isoplethen für jede einzeln oder mehrere gemeinsam der folgenden Pilzspezies bestimmt wird:
a) Pilze, die selbst oder deren Stoffwechselprodukte gesundheitsgefährdend sind und in einem Wohnraum nicht auftreten dürfen,
b) Pilze, die selbst oder deren Stoffwechselprodukte bei längerer Exposition in Räumen gesundheitsgefährdend sind oder ein allergenes Potential besitzen und
c) Pilze, die nicht gesundheitsgefährdend sind, deren Bewuchs aber zu wirtschaftlichem Schaden führt.

5. Verfahren nach einem der drei vorhergehenden Ansprüche, **dadurch gekennzeichnet, dass** eine Schar von Isoplethen für jede einzeln oder mehrere gemeinsam der folgenden Substratgruppen bestimmt wird:
a) Optimaler Nährboden,
b) biologisch verwertbare Substrate,
c) Baustoffe mit porigem Gefüge und
d) Baustoffe, die weder abgebaut werden können noch Nährstoffe enthalten.

6. Verfahren nach einem der vorhergehenden Ansprüche, **dadurch gekennzeichnet, dass** der Wassergehalt der Spore aufgrund des an der Oberfläche der Spore auftretenden Mikroklimas bestimmt wird.

7. Verfahren nach einem der vorhergehenden Ansprüche, **dadurch gekennzeichnet, dass** zur Bestimmung des Wassergehaltes der Spore die Spore als von der Wand unabhängig angenommen wird.

8. Verfahren nach einem der vorhergehenden Ansprüche, **dadurch gekennzeichnet, dass** zur Bestimmung des Wassergehalts der Spore der Durchgang von Wasserdampf als Diffusionsprozess durch eine Sporenwand mit einem vorbestimmten s_{d}-Wert bestimmt wird.

9. Verfahren nach dem vorhergehenden Anspruch, **dadurch gekennzeichnet, dass** der vorbestimmte s_{d}-Wert aus experimentell bestimmten Sporenauskeimungszeiten bestimmt wird.

10. Verfahren nach einem der vorhergehenden Ansprüche, **dadurch gekennzeichnet, dass** zur Bestimmung des Wassergehalts der Spore das osmotische Potential der Spore durch eine Feuchtespeicherfunktion bestimmt wird.

11. Verfahren nach einem der Ansprüche 2 bis 10, **dadurch gekennzeichnet, dass** die Isoplethen experimentell bestimmt werden.

12. Verfahren nach einem der Ansprüche 2 bis 10, **dadurch gekennzeichnet, dass** die Isoplethen mit einem Computer berechnet werden.

13. Verfahren nach einem der vorhergehenden Ansprüche, **dadurch gekennzeichnet, dass** das Verfahren zumindest teilweise mit einem Computer durchgeführt wird.

14. Verwendung eines Verfahrens nach einem der vorhergehenden Ansprüche zur Beurteilung der Schimmelpilzgefahr auf oder in Bauteilen oder sonstigen Gegenständen, wie Lebensmitteln oder Holzwerkstoffen, als Teil einer baulichen Sanierungsmaßnahme, zur Planung der Auslegung und Herstellung von Bauprodukten und Lüftungsanlagen, zur Beurteilung des Lüftungsbedarfs von Gebäuden.

## Claims

1. A method for predicting the formation of mould on an object, for example a component, in that biological germination conditions and/or growth requirements, determined experimentally and/or using a computer, for one or more moulds are compared with the hygrothermal conditions occurring on the object determined experimentally and/or using a computer, and it is determined therefrom whether the germination conditions and/or growth requirements are present on the object, having the following steps:
- determining the temperature curve and relative humidity curve on the object,
- determining the water content of a spore on the object, and
- determining whether the water content of the spore over time reaches or exceeds the water content (limit water content) necessary for germination and/or growth.

2. A method according to the preceding claim, **characterised in that** for determination of the necessary water content at least one system of isopleths is ascertained with respect to germination and/or growth of moulds dependent on temperature and humidity.

3. A method according to the preceding claim, **characterised in that** one system of isopleths is determined for each one individually or a plurality jointly of the following fungal species: a) all fungi, b) fungi which occur in buildings and are hazardous to health.

4. A method according one of the preceding two claims, **characterised in that** one system of isopleths is determined for each one individually or a plurality jointly of the following fungal species:
a) fungi which themselves, or the metabolic products of which, are hazardous to health and must not occur in a residential space,
b) fungi which themselves, or the metabolic products of which, are hazardous to health in the event of longer-term exposure in spaces, or which have allergenic potential, and
c) fungi which are not hazardous to health, but growth of which leads to economic damage.

5. A method according one of the preceding three claims, **characterised in that** one system of isopleths is determined for each one individually or a plurality jointly of the following substrate groups:
a) optimal nutrient medium,
b) biologically exploitable substrates,
c) construction materials having a porous structure, and
d) construction materials which neither can be decomposed nor contain nutrients.

6. A method according to one of the preceding claims, **characterised in that** the water content of the spore is determined based on the microclimate occurring on the surface of the spore.

7. A method according to one of the preceding claims, **characterised in that** for determination of the water content of the spore the spore is assumed to be independent of the wall.

8. A method according to one of the preceding claims, **characterised in that** for determination of the water content of the spore the passage of water vapour as a diffusion process through a spore wall with a predetermined s_{d} value is determined.

9. A method according to the preceding claim, **characterised in that** the predetermined s_{d} value is determined from experimentally-determined spore germination times.

10. A method according to one of the preceding claims, **characterised in that** for determination of the water content of the spore the osmotic potential of the spore is determined by a moisture storage function.

11. A method according to one of Claims 2 to 10, **characterised in that** the isopleths are determined experimentally.

12. A method according to one of Claims 2 to 10, **characterised in that** the isopleths are calculated using a computer.

13. A method according to one of the preceding claims, **characterised in that** the method is carried out at least partly using a computer.

14. Use of a method according to one of the preceding claims for assessing the risk of mould on or in components or other objects, such as foodstuffs or wood materials, as part of a structural remediation measure, for planning the design and production of building products and ventilation systems, for assessing the ventilation requirements of buildings.

## Revendications

1. Procédé pour la prédiction de la formation de moisissures sur un objet, par exemple un composant de construction, dans lequel des conditions de germination biologique et/ou des conditions préalables de croissance, déterminées expérimentalement et/ou à l'aide d'un ordinateur, sont, pour une ou plusieurs moisissures, comparées aux conditions hygrothermiques apparaissant sur l'objet, déterminées expérimentalement et/ou à l'aide d'un ordinateur, et on en déduit si les conditions de germination et/ou les conditions préalables de croissance sont présentes sur l'objet, comportant les étapes suivantes :
- détermination de l'évolution de la température et de l'humidité relative sur l'objet,
- détermination de la teneur en eau d'une spore sur l'objet et
- détermination si la teneur en eau de la spore atteint ou dépasse, au cours du temps, la teneur en eau nécessaire à la germination et/ou à la croissance (teneur en eau limite).

2. Procédé selon la revendication précédente, **caractérisé en ce que**, pour la détermination de la teneur nécessaire en eau, on détermine un réseau d'isoplèthes concernant la germination et/ou la croissance de moisissures en fonction de la température et de l'humidité.

3. Procédé selon la revendication précédente, **caractérisé en ce qu'**un réseau d'isoplèthes est déterminé pour les espèces de champignons suivantes, prises soit individuellement, soit à plusieurs en commun : a) tous les champignons, b) les champignons qui apparaissent dans les bâtiments et qui mettent la santé en danger.

4. Procédé selon l'une des deux revendications précédentes, **caractérisé en ce qu'**on détermine un réseau d'isoplèthes pour les espèces de champignons suivantes, prises soit individuellement, soit à plusieurs en commun :
a) les champignons qui, par eux-mêmes, ou leurs métabolites, présentent un danger pour la santé et ne doivent pas apparaître dans un local d'habitation,
b) les champignons qui, par eux-mêmes ou leurs métabolites présentent dans le cas d'une exposition prolongée un danger pour la santé dans les locaux, ou possèdent un potentiel allergène, et
c) les champignons qui ne présentent pas de danger pour la santé, mais dont l'infestation conduit à des dommages économiques.

5. Procédé selon l'une des trois revendications précédentes, **caractérisé en ce qu'**on détermine un réseau d'isoplèthes pour les groupes de substrats suivants, soit individuellement, soit à plusieurs en commun :
a) les sols nutritifs optimaux,
b) les substrats pouvant subir une exploitation biologique,
c) les matériaux de construction à structure poreuse, et
d) les matériaux de construction qui ne peuvent être dégradés, ni ne contiennent de nutriments.

6. Procédé selon l'une des revendications précédentes, **caractérisé en ce que** la teneur en eau de la spore est déterminée sur la base du microclimat apparaissant sur la surface de la spore.

7. Procédé selon l'une des revendications précédentes, **caractérisé en ce que**, pour la détermination de la teneur en eau de la spore, on prend en compte la spore indépendamment de sa paroi.

8. Procédé selon l'une des revendications précédentes, **caractérisé en ce que**, pour la détermination de la teneur en eau de la spore, on détermine, par une valeur S_{d} prédéterminée, le passage de la vapeur d'eau, par un processus de diffusion, à travers une paroi sporale.

9. Procédé selon la revendication précédente, **caractérisé en ce que** la valeur S_{d} prédéterminée est déterminée à partir de temps de germination de spores déterminés par voie expérimentale.

10. Procédé selon l'une des revendications précédentes, **caractérisé en ce que**, pour la détermination de la teneur en eau de la spore, on détermine le potentiel osmotique de la spore par une fonction d'accumulation d'humidité.

11. Procédé selon l'une des revendications 2 à 10, **caractérisé en ce que** les isoplèthes sont déterminés expérimentalement.

12. Procédé selon l'une des revendications 2 à 10, **caractérisé en ce que** les isoplèthes sont calculés à l'aide d'un ordinateur.

13. Procédé selon l'une des revendications précédentes, **caractérisé en ce que** le procédé est au moins partiellement mis en œuvre à l'aide d'un ordinateur.

14. Utilisation d'un procédé selon l'une des revendications précédentes pour évaluer le risque de moisissures sur ou dans des composants de construction ou d'autres objets tels que des produits alimentaires ou des matériaux à base de bois, en tant que partie d'une opération de rénovation de bâtiments, pour la planification de la conception et de la fabrication de produits de construction et d'installations d'aération, pour l'évaluation des besoins en aération de bâtiments.
